(19) Europäisches Patentamt
European Patent Office
Office européen des brevets

(11) Veröffentlichungsnummer : **0 061 711**
**B1**

(12) # EUROPÄISCHE PATENTSCHRIFT

(45) Veröffentlichungstag der Patentschrift : **13.06.84**

(51) Int. Cl.³ : **C 07 B 23/00**, C 07 F 5/02, A 61 K 41/00, A 61 K 31/69

(21) Anmeldenummer : **82102450.2**

(22) Anmeldetag : **24.03.82**

(54) **10B-carboxy-aminoborane, Verfahren zur Herstellung derselben sowie ihre Verwendung als Arzneimittel zur Neutroneneinfangtherapie.**

(30) Priorität : **27.03.81 DE 3112170**

(43) Veröffentlichungstag der Anmeldung : **06.10.82 Patentblatt 82/40**

(45) Bekanntmachung des Hinweises auf die Patenterteilung : **13.06.84 Patentblatt 84/24**

(84) Benannte Vertragsstaaten : **AT BE CH DE FR GB IT LI NL SE**

(56) Entgegenhaltungen :
**JOURNAL OF THE AMERICAN CHEMICAL SOCIETY, Band 98.18, 1. September 1976, Seiten 5702-5703**

(73) Patentinhaber : **Mückter, Heinrich, Dr.
Am Chorusberg 51
D-5100 Aachen (DE)**

**Dallacker, Franz, Prof. Dr.
Weberstrasse 5
D-5120 Herzogenrath (DE)**

**Müllners, Wilhelm
Breite Strasse 36
D-5142 Hückelhoven (DE)**

(72) Erfinder : **Mückter, Heinrich, Dr.
Am Chorusberg 51
D-5100 Aachen (DE)**
Erfinder : **Dallacker, Franz, Prof. Dr.
Weberstrasse 5
D-5120 Herzogenrath (DE)**
Erfinder : **Müllners, Wilhelm
Breite Strasse 36
D-5142 Hückelhoven (DE)**

(74) Vertreter : **Werner, Hans-Karsten, Dr. et al
Deichmannhaus am Hauptbahnhof
D-5000 Köln 1 (DE)**

**EP 0 061 711 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der vorliegenden Erfindung sind carboxy-aminoborane der Formel I

$$R_3NBH_2—COOH \quad (I)$$

in der R Wasserstoff oder eine Methylgruppe darstellt mit einem $^{10}$B-Gehalt von größer als 80 %, vorzugsweise größer als 90 %, ihre physiologisch verträglichen Salze sowie ein Verfahren zu ihrer Herstellung und die Verwendung als Arzneimittel, vorzugsweise zur Neutroneneinfangtherapie.

Es ist bekannt, daß das Borisotop $^{10}$B mit Neutronen reagiert, wobei Lithiumisotop Li, ein α-Teilchen sowie eine definierte Energiemenge freigesetzt wird. Diese Reaktion folgt folgendem Schema:

$$_5{}^{10}B + n \rightarrow {}_3{}^7Li + \alpha + 2{,}78\ MeV.$$

Findet diese Reaktion in vivo statt, wirken das freiwerdende α-Teilchen der $^7$Lithium-Kern und die freiwerdende Energie destruierend über eine Strecke von etwa 10 μ, was etwa dem Durchmesser einer Zelle entspricht. F.M. Waterman, F.T. Kuchnir, L.S. Skaggs, D.K. Bewly, B.C. Page und F.H. Attix haben diese Reaktion zur Behandlung von Tumorzellen untersucht; vgl. Phys. Med. Biol., 1978, Vol. 23, No. 4, S. 592-602. Die Probleme dieser Therapie bestehen einmal darin, Neutronen mit geeigneter Energie zu erzeugen, welche die vorhandenen Tumore im Körper mit genügender Intensität erreichen und zum anderen $^{10}$Bor-Verbindungen zu finden, welche sich zum Zeitpunkt der Neutronenbestrahlung in möglichst hoher Konzentration in den Tumorzellen anreichern und im übrigen für die normalen Zellen des menschlichen Organismus möglichst wenig toxisch sind. Für derartige Untersuchungen bei Tier und Mensch wurden bisher vor allem Natriumpentaborat und Mercaptoundecahydrodecaborat untersucht. Diese Substanzen sind jedoch relativ toxisch und sie besitzen daher nur eine begrenzte therapeutische Wirkung.

Es wurde jetzt gefunden, daß $^{10}$B-carboxy-aminoborane mit einem $^{10}$B-Gehalt von größer als 80 %, vorzugsweise größer als 90 % (Atom %), in hervorragender Weise für die Neutroneneinfangtherapie geeignet sind und zu erstaunlich guten und nicht vorhersehbaren therapeutischen Ergebnissen führen.

N-Trimethyl- B-carboxy-aminoboran wurde neben vielen weiteren Borverbindungen von Spielvogel u. a. synthetisiert und untersucht; vgl. J. Am. Chem. Soc., 98 : 18, S. 5702, Journal Pharmaceutical Sciences, Vol. 68, Nr. 6, 1979, S. 685 und Journal of Pharmaceutical Sciences, Vol. 69, Nr. 9, S. 1025. Als Wirksamkeit werden Antitumorwirkung, hypolipidämische Wirksamkeit und schließlich entzündungshemmende Aktivität beschrieben. Diese pharmakologischen Eigenschaften bleiben beim Ersatz des $^{11}$Bor-Atoms durch das $^{10}$Bor-Atom erhalten, genauso wie die geringe Toxizität. Wird die erfindungsgemäße Substanz bei der Neutroneneinfangtherapie eingesetzt, kommt es zu einer nicht vorhersehbaren synergistischen Wirkung, die zu ungewöhnlichen Therapieerfolgen führt.

Die geringe Toxizität der erfindungsgemäß verwendeten Substanz ermöglicht eine so hohe Dosierung, daß sowohl eine begrenzte Eigenwirkung zum Tragen kommt als auch insbesondere eine ausreichende Erhöhung der $^{10}$Bor-Konzentration in den erkrankten Zellen erreicht wird. So wurde festgestellt, daß die Toxizität bei der Maus i. p. eine $DL_{50}$ um 2 000 mg/kg ergibt. Pharmakologisch wurde festgestellt, daß N-Trimethyl-$^{10}$B-carboxyaminoboran den Einbau von $^{125}$Jod-desoxyuridin bei der gesunden Maus senkt. Weiterhin senkt es den Einbau von $^{125}$Jod-desoxyuridin in das Sarkom 180 der Maus. Eine gleichzeitige Behandlung mit γ-Strahlen wird durch die erfindungsgemäße Substanz nicht beeinträchtigt, jedoch wird das Tumorwachstum durch eine Kombination von N-Trimethyl-$^{10}$B-carboxyaminoboran und γ-Strahlen positiv beeinflußt. Die Applikation von N-Trimethyl-$^{10}$B-carboxy-aminoboran führt beim Sarkom 180 der Maus zu einer Verkleinerung des Tumors. Durch gleichzeitige Behandlung mit thermischen Neutronen wird das Tumorvolumen wesentlich stärker verkleinert als durch die Einzelkomponenten.

Besonders eindrucksvoll ist die Veränderung der Überlebenszeiten der Sarkom 180 tragenden NMRI-Mäuse. Während 40 Tage nach Beginn der Behandlung von den unbehandelten und den nur mit Neutronen behandelten Tieren ca. 90 % am Tumor gestorben sind, überleben zu diesem Zeitpunkt ca. 40 % der nur mit N-Trimethyl-$^{10}$B-carboxy-aminoboran und ca. 75 % der mit dieser Substanz und Neutronen behandelten Tiere.

Ein Vergleich mit Natriumpentaborat und Neutronen zeigt, daß nur 20 bis 30 % der Sarkom-Tiere überleben.

An keinem der überlebenden Tiere wurden lokale oder systemische Schäden beobachtet. Diese pharmakologischen Befunde wurden zunächst noch mit einem N-Trimethyl- B-carboxy-aminoboran erhalten, welches nur 20 % $^{10}$Bor enthielt. Durch Steigerung des $^{10}$Bor-Gehalts auf 80 bis 90 % erhöht sich die Antitumorwirkung durch Neutroneneinfang um das 4 bis 5-fache:

C-57 BL/6j Mäuse mit Adenocarcinom EO 771 werden in drei Gruppen aufgeteilt:

Gruppe 1 dient als Kontrolle

Gruppe 2 wird 6 Minuten mit thermischen Neutronen bestrahlt

Gruppe 3 erhält 10 mg N-Trimethyl-$^{10}$B-carboxyaminoboran intraperitonal (i. p.) und wird nach 6 Stunden — dem Zeitpunkt der optimalen Anreicherung im Tumorgewebe — für 6 Minuten mit thermischen Neutronen bestrahlt. Während die unbehandelten Kontrollen und nur mit Neutronen behandelten Tiere ein stetiges Wachstum des

Tumors zeigen, nimmt der Tumor in Gruppe 3 nach der kombinierten Behandlung an Größe ab und ist nach etwa 7 Tagen auf ein Minimum reduziert.

Das bisher für die Neutroneneinfangtherapie verwendete Natriummonomercaptoundecahydrodecaborat ist etwa 5mal toxischer als die erfindungsgemäße Verbindung. Sie kann somit nur in relativ geringeren Mengen eingesetzt werden und besitzt keine eigene Antitumorwirkung.

Eine weitere interessante Anwendungsmöglichkeit der erfindungsgemäßen Substanz besteht in der Therapie von Arthritis, da bekannt ist, daß in schweren Fällen von Arthritis auch zytostatisch wirkende Substanzen systemisch und lokal mit guter Wirksamkeit eingesetzt werden können, allerdings mit dem Risiko einer hohen Toxizität für den Gesamtorganismus. Diese Therapie beruht auf der Beobachtung, wonach die Synovia, die Schleimhaut in den Gelenken, im Stadium der schweren chronischen Arthritis biologisch dem Verhalten von Tumorzellen ähnelt. Bei intraartikulärer Applikation von N-Trimethyl-$^{10}$-B-carboxy-aminoboran und gezielter Neutronenbestrahlung der erkrankten Schleimhaut der Gelenke ist mit vergleichbaren Erfolgen wie bei der Behandlung von Tumoren zu rechnen. Hinzu kommt noch die eigene antiarthritische Wirkung der Substanz.

Applikation und Dosierung hängen in sehr starkem Maße von Art und Lokalisation des Tumors ab. Die Applikation des N-Trimethyl-$^{10}$B-carboxyaminoborans kann intravenös erfolgen, da es ausreichend wasserlöslich und verträglich ist. Anstelle einer einmaligen hohen Dosierung und anschliessenden einmaligen Neutronenbehandlung kann die Substanz auch an mehreren Tagen hintereinander in geringerer Dosierung verabfolgt und die Neutroneneinfangtherapie wiederholt werden. Es werden dabei Einzeldosen von 20 bis 200 mg/kg zur Anwendung kommen. Besonders hohe $^{10}$Bor-Konzentrationen werden im Tumorgewebe erreicht, wenn die Substanz intraarteriell in die versorgende Arterie oder intratumoral, d. h. in den Tumor, injiziert wird.

Die Substanz reichert sich im Tumorgewebe an, so werden 6 bis 12 Stunden nach einer intravenösen Injektion der Substanz im Tumor 2 bis 3mal höhere Bor-Konzentrationen gemessen als im Blut. Die selektive Anreicherung (drugtargeting) läßt sich über eine Bindung an tumoraffine Trägersubstanzen (carrier) wie Enzyme, Antikörper, Haematoporphyrine u. a. noch verstärken. Anstelle des N-Trimethyl-$^{10}$B-carboxyaminoborans können auch seine physiologisch verträglichen Salze verwendet werden. Vorzugsweise werden als Salze das Natrium- oder Calciumsalz verwendet.

Noch bessere therapeutische Ergebnisse in Verbindung mit Neutronen werden erzielt durch Verwendung von Ammoniak-$^{10}$B-carboxyboran. Diese Verbindung enthält 13,61 % Bor und hat eine sehr niedrige Toxizität ($DL_{50}$ i. p. Maus um 2 500 mg/kg). Diese Substanz ist durch Austausch der Trimethylaminogruppe von erfindungsgemäßem N-Trimethyl-$^{10}$B-carboxyaminoboran durch die Aminogruppe beispielsweise durch Ammoniak unter Druck bei Raumtemperatur herstellbar; vgl. Am. Chem. Soc. *102,* 6 344 (1980).

Gewünschtenfalls wird anschliessend in physiologisch verträgliche Salze überführt, beispielsweise durch Zusatz einer äquivalenten Menge von Natronlauge oder Natriumcarbonat.

In den folgenden Beispielen ist die Herstellung der erfindungsgemäßen Substanzen näher beschrieben.

Beispiel 1 (N-Trimethyl-$^{10}$B-carboxyaminoboran)

Zu einer intensiv gerührten Suspension von 6,5 g (0,175 Mol) Natrium-$^{10}$B-borhydrid mit einem $^{10}$B-Gehalt von 90 % und 100 ml Tetrahydrofuran, die sich in einem Wasserbad befindet, tropft man bei Raumtemperatur innerhalb von 10 Minuten 8,5 g (0,315 Mol) frisch destillierte Blausäure gelöst in 40 ml wasserfreiem Tetrahydrofuran. Zwei Stunden nach beendeter Zugabe erwärmt man allmählich zum Sieden und erhitzt zwei Stunden unter Rückfluß. Nach 12 Stunden Rühren bei Raumtemperatur erhitzt man kurz zum Sieden und leitet zur Entfernung überschüssiger Blausäure einen starken Stickstoffstrom durch die sich abkühlende Lösung. Man filtriert ab, engt am Rotationsverdampfer ein und erhitzt im Vakuum 4 bis 6 Stunden auf 60 °C (Badtemperatur). Man erhält so 9,2 g (86 % der Theorie) eines hellgelben, stark hygroskopischen Pulvers von Natrium-cyano-$^{10}$B-borhydrid.

Unter Kühlen mit Eis/Wasser gibt man zu einer Suspension von 15,7 g (0,164 Mol) Trimethylaminhydrochlorid und 150 ml wasserfreiem Tetrahydrofuran 8,5 g (0,137 Mol) des Natriumcyano-$^{10}$B-borhydrids. Nach Entfernen des Eisbades erhitzt man im Verlaufe von 2 Stunden zum Sieden und setzt das Erhitzen 95 Stunden bei einer Badtemperatur von 80 bis 85 °C fort. Man filtriert die abgekühlte Lösung und wäscht dreimal mit je 20 ml Tetrahydrofuran. Man engt das Filtrat bei 35 °C am Rotationsverdampfer zur Trockne ein, extrahiert mit 50 ml Methylenchlorid, filtriert und wäscht den Rückstand 3mal mit je 10 ml Methylenchlorid. Nach Abdestillieren des Lösungsmittels erhält man 12,0 g (90 % der Theorie) des N-Trimethyl-$^{10}$B-cyano-aminoborans als hellgelbe Substanz mit einem Schmelzbereich von 56 bis 63 °C.

Zur Reinigung löst man in Methylenchlorid, wäscht mehrmals mit gesättigter Kochsalzlösung, trocknet über Magnesiumsulfat und kristallisiert den Destillationsrückstand aus Benzin/Tetrahydrofuran (2 : 1) um. Man erhält so farblose Nadeln vom Schmelzpunkt 64 bis 66 °C.

Zu einer Lösung von 27 g (0,14 Mol) Triethyloxoniumtetrafluoroborat und 20 ml Methylenchlorid, die sich in einem 100 ml Schliffkolben befindet, gibt man unter Eiskühlung 10,0 g (0,103 Mol) des rohen N-Trimethyl-$^{10}$B-cyanoaminoborans. Nach 3 bis 5 Stunden tritt bei

Raumtemperatur Phasentrennung und beginnende Kristallisation ein. Man läßt das Reaktionsgemisch 2 Tage bei Raumtemperatur stehen, kühlt kurz auf − 75 °C ab, filtriert und wäscht den Rückstand 3mal mit je 2 bis 3 ml Methylenchlorid. Man erhält so 19,9 g (91 % der Theorie) N-Trimethyl-$^{10}$B-cyano-aminoboran-N'-ethylnitrilium-tetrafluoroborat als schwach gelb gefärbtes Produkt mit einem Schmelzintervall von 90-130 °C. Gewünschtenfalls wird durch Umkristallisation aus Methylenchlorid wie oben beschrieben, weiter gereinigt. Man erhält farblose Kristalle vom Schmelzpunkt 146-149 °C.

18,7 g (0,088 Mol) des rohen N-Trimethyl-$^{10}$B-cyano-aminoboran-N'-ethylnitrilium-tetrafluoroborats versetzt man mit 18 ml (1 Mol) Wasser, läßt 30 Stunden bei Raumtemperatur stehen und setzt gewünschtenfalls zur Beschleunigung der Kristallisation Impfkristalle zu. Nach 4 Tagen kühlt man mit Eis/Wasser, filtriert und wäscht mit kaltem Wasser nach. Das Filtrat engt man bei Raumtemperatur auf ca. 20 ml ein und filtriert erneut. Die vereinigten Fraktionen (ca. 7,2 g) versetzt man mit 9 ml siedend heißem Wasser, erhitzt bis zur Lösung und stellt schnell zur Kristallisation in Eis/Wasser. Man erhält 6,2 g (61 % der Theorie) N-Trimethyl-$^{10}$B-carboxy-aminoboran als farblose Kristalle vom Zersetzungspunkt 126 °C. Der Schmelzpunkt ist von der Aufheizgeschwindigkeit und der Kristallgröße abhängig. Der höchste ermittelte Schmelzpunkt lag bei 148 °C.

Der $^{10}$B-Gehalt wurde massenspektroskopisch zu 90,6 ± 0,5 % (Atom %) des Gesamtborgehaltes ermittelt.

Beispiel 2 (Ammoniak-$^{10}$B-carboxyboran)

In einen mit Trockeneis/Isopropylalkohol gekühlten Stahlautoklaven gibt man zu 15 g (0,129 mol) N-Trimethyl-amino-$^{10}$B-carboxyboran (hergestellt gemäß Beispiel 1) 220 ml (ca. 7 mol) auf − 75 °C gekühltes, flüssiges Ammoniak. Man verschließt und läßt 18 Tage bei Raumtemperatur stehen. Dann kühlt man wieder auf − 75 °C, öffnet und gießt die Lösung in einen Kolben. Man vertreibt das Ammoniakgas im Stickstoffstrom und legt schließlich 1 Std. Wasserstrahlvakuum an. Den Rückstand erhitzt man in 250 ml Chloroform 3 Std. unter Rückfluß. Man filtriert heiß ab und wäscht den Filterrückstand mehrmals mit heißem Chloroform.

Es verbleiben 5 g (52 % der Theorie) eines Rohproduktes, das bei 110 °C zu erweichen beginnt und sich bei 115-118 °C zersetzt. Zur Reinigung gibt man zu 1 g Rohprodukt 2 ml 40 °C warmes Wasser, kühlt schnell auf 0 °C, saugt ab und wäscht mit einigen Tropfen kaltem Wasser. Man erhält 500 mg farbloses Pulver Zers.-P. 116-118 °C.

Durch Einengen des Filtrats im Wasserstrahlvakuum erhält man 120 mg farblose Kristalle Zers.-P. 119 °C. $CH_6BNO_2$(74.165)

Ber.: C 16.14 H 8.15 B 13.61 %
Gef.: C 16.32 H 8.07

## Ansprüche

1. $^{10}$B-carboxy-aminoborane der Formel I

$$R_3NBH_2{-}COOH \qquad (I)$$

in der R Wasserstoff oder eine Methylgruppe darstellt mit einem $^{10}$B-Gehalt von mehr als 80 %, vorzugsweise mehr als 90 % sowie ihre physiologisch verträglichen Salze.

2. Verfahren zur Herstellung der $^{10}$B-carboxy-aminoborane der Formel I sowie ihre physiologisch verträglichen Salze mit einem $^{10}$B-Gehalt von mehr als 80 %, vorzugsweise mehr als 90 %, dadurch gekennzeichnet, daß man N-Trimethyl-$^{10}$B-cyano-aminoboran der Formel II

$$(CH_3)_3NBH_2{-}CN$$

mit Triethyloxoniumtetrafluoroborat zum N-Trimethyl-$^{10}$B-cyano-aminoboran-N'-ethylnitrilium-tetrafluoroborat umsetzt und anschließend hydrolysiert ggf. mit Ammoniak die Trimethylaminogruppe in die Aminogruppe überführt und die so erhaltenen $^{10}$B-Carboxyaminoborane gewünschtenfalls in die physiologisch verträglichen Salze überführt.

3. Verwendung von $^{10}$B-carboxy-aminoboranen der Formel I sowie ihrer physiologisch verträglichen Salze mit einem $^{10}$B-Gehalt von größer als 80 %, vorzugsweise größer als 90 % zur Herstellung von Arzneimitteln zur Neutroneneinfangtherapie.

4. Arzneimittel zur Neutroneneinfangtherapie, gekennzeichnet durch einen Gehalt von $^{10}$B-carboxy-aminoboranen der Formel I mit einem $^{10}$B-Gehalt von größer als 80 %, vorzugsweise größer als 90 % sowie ihrer physiologisch verträglichen Salze.

## Claims

1. $^{10}$B-carboxy-amino boranes of the formula I

$$R_3NBH_2{-}COOH \qquad (I)$$

wherein R represents a hydrogen atom or a methyl group, having a $^{10}$B content of more than 80 %, preferably of more than 90 %, and their physiologically compatible salts.

2. A method for preparing the $^{10}$B-carboxy-amino boranes of the formula I and the physiologically compatible salts thereof having a $^{10}$B content of more than 80 %, preferably of more than 90 %, characterized in that N-trimethyl-$^{10}$B-cyano-amino borane of the formula II

$$(CH_3)_3NBH_2{-}CN \qquad (II)$$

is reacted with triethyloxonium tetrafluoroborate to give N-trimethyl-$^{10}$B-cyano-amino borane N'-ethylnitrilium tetrafluoroborate, followed by hydrolysis, and the trimethylamine group is optionally converted into the amine group by means of

ammonia, with converting the $^{10}B$-carboxy-amino boranes having been thus obtained into physiologically compatible salts if desired.

3. Use of $^{10}B$-carboxy-amino boranes of the formula I and of the physiologically compatible salts thereof having a $^{10}B$ content of more than 80 %, preferably of more than 90 %, for preparing drugs for neutron capture therapy.

4. Drug for neutron capture therapy, characterized in that it contains a $^{10}B$-carboxy-amino borane of the formula I having a $^{10}B$ content of more than 80 %, preferably of more than 90 %, and/or the physiologically compatible salts thereof.

**Revendications**

1. Des $^{10}B$-carboxyaminoboranes de formule I

$$R_3NBH_2{-}COOH \qquad (I)$$

dans laquelle R représente de l'hydrogène ou un groupe méthyle, avec une teneur en $^{10}B$ supérieure à 80 %, de préférence supérieure à 90 % ainsi que leurs sels physiologiquement acceptables.

2. Procédé de préparation des $^{10}B$-carboxyaminoboranes de formule I ainsi que leurs sels physiologiquement acceptables avec une teneur en $^{10}B$ supérieure à 80 %, de préférence supérieure à 90 %, caractérisé en ce que l'on fait réagir du N-triméthyl-$^{10}B$-cyanoaminoborane de formule II

$$(CH_3)_3NBH_2{-}CN$$

avec du tétrafluoborate de triéthyloxonium pour obtenir du tétrafluoborate de N-triméthyl-$^{10}B$-cyanoaminoborane-N'-éthylnitrilium et hydrolyse consécutivement, que l'on transforme éventuellement le groupe triméthylamino avec de l'ammoniac en le groupe amino et que l'on convertit au besoin les $^{10}B$-carboxyaminoboranes ainsi obtenus en les sels physiologiquement acceptables.

3. Utilisation des $^{10}B$-carboxyaminoboranes de formule I ainsi que de leurs sels physiologiquement acceptables avec une teneur en $^{10}B$ supérieure à 80 %, de préférence supérieure à 90 %, pour la préparation de médicaments pour la thérapeutique par capture de neutrons.

4. Médicament pour le traitement par capture de neutrons, caractérisé en ce qu'il contient des $^{10}B$-carboxyaminoboranes de formule I avec une teneur en $^{10}B$ supérieure à 80 %, de préférence supérieure à 90 % ainsi que leurs sels physiologiquement acceptables.